# EUROPEAN PATENT SPECIFICATION

(11) **EP 0 646 182 B1**
(45) Date of publication and mention of the grant of the patent: **28.08.1996**
(21) Application number: 93916537.9
(22) Date of filing: 14.06.1993
(51) Int. Cl.: C12Q 1/68, G01N 33/574

(54) **PROCESS FOR DETECTION OF NEOPLASTIC DISEASE**
VERFAHREN ZUR DETEKTION NEOPLASTISCHER ERKRANKUNGEN
PROCEDE DE DETECTION DES MALADIES NEOPLASIQUES

(30) Priority: 18.06.1992 US 900646
(43) Date of publication of application: 05.04.1995
(73) Proprietor: THE WHITTIER INSTITUTE FOR DIABETES AND ENDOCRINOLOGY, La Jolla, CA 92037 (US)
(72) Inventor: NOVA, Michael, Philip, La Jolla, CA 92037 (US); GONZALEZ, Ana-Maria, Del Mar, CA 92014 (US); BAIRD, J., Andrew, San Diego, CA 92122 (US)
(74) Representative: Goldin, Douglas Michael
(86) International application number: PCT/US93/05703
(87) International publication number: WO 94/00599

(56) References cited:
- EP-A- 0 341 904
- EP-A- 0 387 777
- WO-A-91/00916
- WO-A-91/11459
- NUCLEIC ACIDS RESEARCH vol. 18, no. 7 , 1990 , ARLINGTON, VIRGINIA US page 1906 A. ISACCHI ET AL. cited in the application
- EMBO JOURNAL vol. 10, no. 6 , 1991 , EYNSHAM, OXFORD GB pages 1347 - 1354 J. PARTANEN ET AL. cited in the application
- ONCOGENE 4 (7). 1989. 915-922. CODEN: ONCNES ISSN: 0950-9232 THEILLET C ET AL 'AMPLIFICATION OF FGF -RELATED GENES IN HUMAN TUMORS POSSIBLE INVOLVEMENT OF HST IN BREAST CARCINOMAS.'
- BIOCHIMICA ET BIOPHYSICA ACTA vol. 1089 , 1991 pages 244 - 246 M. SENO ET AL. 'Two cDNAs encoding novel human FGF receptor' cited in the application
- 30TH ANNUAL MEETING OF THE AMERICAN SOCIETY OF DERMATOPATHOLOGY, SAN FRANCISCO, CALIFORNIA, USA, DECEMBER 2-4, 1992. J CUTANEOUS PATHOL 19 (6). 1992. 544. CODEN: JCUPBN ISSN: 0303-6987 NOVA M P ET AL 'THE FIBROBLAST GROWTH FACTOR RECEPTOR AS A DIAGNOSTIC AGENT FOR MALIGNANT MELANOMA.'
- ONCOGENE vol. 7 , November 1992 pages 2303 - 2313 D. BECKER ET AL.
- J.Cell Biol. Vol.107 p.1611-1619 (1988)
- Oncogene Research Vol.3 p.177-186 (1988)
- NUCLEIC ACIDS RESEARCH, vol. 18, no. 7, 1990, Arlington, VA (US); A. ISACCHI et al., p. 1906/
- THE EMBO JOURNAL, vol. 10, no. 6, 1991, Eynsham, Oxford (GB); J. PARTANEN et al., pp. 1347-1354/
- ONCOGENE, vol. 4, no. 7, 1989, CODEN, ONCNES ISSN: 0950-9232; C. THEILLET et al., pp. 915-922/
- BIOCHIMICA ET BIOPHYSICA ACTA, vol. 1089, 1991; M. SENO et al., pp. 244-246/
- JOURNAL OF CUTANEOUS PATHOLOGY, vol. 19, no. 6, 30th Annual Meeting of the American Society of Dermatopathology, San Francisco, CA (US), 02-04 December 1992; M.P. NOVA et al., p. 544/
- ONCOGENE, vol. 7, November 1992; D. BECKER et al., pp. 2303-2313/

## Description

### FIELD OF THE INVENTION

This invention relates to processes for diagnosing cancer. In a more specific aspect it relates to methods for detecting melanoma.

### BACKGROUND OF THE INVENTION

Despite improvements in surgical and radiological procedures and an increasingly large arsenal of drugs for treating cancer, cures for the many malignant neoplastic diseases remain elusive. The strides that have been made in reducing mortality have come largely from education programs that have heightened public awareness of the need for routine physical examination and improved techniques for detecting the presence of neoplastic disease at a sufficiently early stage to provide a favorable prognosis. Such improved techniques include immunoassays for tumor associated antigens, such as carcinoembryonic antigen (CEA) and alphafetoprotein (AFP), and nucleic acid hybridization techniques. Nucleic acid hybridization can be used, for example, to diagnose the presence of chromosomal translocations associated with certain forms of cancer, such chromosomal translocations associated with chronic myelogenous leukemia (CML). Successful techniques, however, are rare.

Accordingly, there remains a pressing need for better cancer diagnostics. For example, if detected before metastasis, malignant melanoma is an excellent example of a virulent cancer that is treatable by surgical removal with a high rate of success. After metastasis, however, the five year mortality rate using available chemotherapy and radiotherapy techniques is a shocking 97-99% (6,500 deaths in the United States in 1991). The shortcomings of modern treatments for melanoma take on heightened significance when it is appreciated that the incidence of this once relatively rare cancer is increasing annually at a rate of 4-10%, with 32,000 new cases in the United States in 1991.

Early detection of melanoma is complicated because many melanomas presented for diagnosis are either atypical in appearance in that they appear identical to non-melanocytic lesions or are obscured by inflammation. Some melanomas are completely amelanotic (non-pigmented) and others are histologically similar to benign melanocytic growths. Thus, using classical histological techniques, it is frequently impossible to determine whether a lesion is actually a melanoma or whether it is a benign melanocytic growth or other type of carcinoma, for example a sarcoma.

In many cases, particularly those in which histological examination of biopsy material is used, it remains difficult to classify the kind of tumor which may be present. It is also difficult to differentiate malignancies from benign tumors and other non-malignant tissue abnormalities. Of perhaps even greater consequence to the patient, the techniques that are available lack in sensitivity so that early stage disease, the most opportune time for therapeutic intervention, is often missed entirely.

Thus, there is a need for methods for detection neoplasms, including malignant melanoma. In particular there is a need for relatively simple methods that can be used to quickly assess the malignancy of biopsied lesions or to test urine, blood or other body fluids for the a marker indicative of neoplastic disease. Thus far, except in the few instances noted above, reliable means for performing such methods have proved elusive. For example, there have been attempts to develop immunohistochemical tests for diagnosing neoplastic. The resulting tests, however,are often inconclusive, arbitrary and, generally, not diagnostically useful (see, e.g., Gaffey (1991) Amer. J. Clin. Path. 96:679-680). In fact, the search for a reproducible "cancer stain" has been deemed "a Holy Grail" for pathologists (see, Gaffey at page 680).

As the foregoing demonstrates, there clearly exists a pressing need for better techniques to detect and classify neoplastic diseases like melanoma. Therefore, it is an object herein to provide methods for early diagnosis of neoplastic disease, including melanoma. In particular, it is an object herein to provide immunohistochemical methods and other methods for the detection of markers in biopsied tissue samples, urine, blood and other body fluids that are indicative of neoplastic disease.

### SUMMARY OF THE INVENTION

Fibroblast growth factor (FGF) is an almost ubiquitous protein that is highly multifunctional and present in many tissues. It has generally been thought to act in vivo on the same cell types in which it acts in vitro. As a corollary, it has been assumed that FGF receptors are present in vivo on the same tissues on which FGF acts in vitro. As described herein, however, contrary to predictions made from the observations made on cells in culture, the receptor for FGF is differentially expressed in vivo. For example, as described herein, certain neoplastic tissues and cells after injury have elevated levels of receptor when compared to the low to absent levels in non-malignant quiescent tissue. Consequently, as shown herein, the elevated levels of expression of the receptor gene by select neoplasms can be used in the diagnosis of such neoplasms.

Accordingly, methods for detecting the presence of neoplastic disease detecting elevated levels of expression of are provided. Such elevated levels may be detected by any suitable method. For example, they can be detected determining the presence of the receptor or its mRNA in cellular material or by determining the presence of the receptor in body fluids as a result of sloughing from tumor cells. Thus, the methods involve the detection of a product associated with expression of a gene, such as mRNA, that encodes a protein receptor for fibroblast growth factor (FGF) or detection of FGF receptor in cells, tissues, or a body fluid, such as, for example, blood, serum or urine.

When an assay is performed directly on suspect cells, the target product can be either the messenger RNA (mRNA) transcription product of the gene encoding the receptor, cDNA prepared from the mRNA or the receptor protein. Techniques for detecting mRNA that employ nucleic acid hybridization are useful for directly assaying suspect cells. The hybridization methods may be performed in situ, on mRNA isolated from suspect cells, or on cDNA prepared from the mRNA. Such methods employ nucleic acid probes having a nucleotide sequence that is sufficiently complementary to that of the receptor and of a sufficient length to selectively hybridize to the mRNA or cDNA encoding the receptor to form detectable hybrids. The probe is allowed to bind with the target nucleic acid in a manner that permits detection of the resulting duplex using art established techniques.

In the case of body fluids and histopathologic analysis, the target expression product is preferably the receptor protein. Detection of the receptor protein can be accomplished by any technique which is specific for the protein. For example, immunological techniques employing antibodies to the receptor or a portion thereof that form a detectable antibody/receptor complex can be used.

The assays herein may be used to detect any neoplasm that results in higher levels of FGF receptor protein or FGF receptor-encoding mRNA in a cell, tissue or body fluid. Such neoplasms include, but are not limited to certain breast cancers, ovarian cancer, prostate hyperplasia, prostate carcinoma, astrocytoma, glioblastoma and malignancies and diseases that are subsequently shown to be associated with elevated FGF receptor expression. For example, as demonstrated below, the detection of a specific FGF receptor or mRNA that encodes the receptor that is associated with malignant melanoma is particularly advantageous since they are increased in amounts when compared to normal or benign lesions. Accordingly, the assay is described below with particular reference to its use for detecting malignant melanoma.

In preferred embodiments, assays that distinguish suspect lesions that are malignant melanoma from those that are not are provided, Such assays provide a relatively simple staining procedure for diagnosing early stage malignant melanoma.

Kits for use in performing the assays for detection of neoplasms are also provided.

### DETAILED DESCRIPTION OF THE INVENTION

### Definitions

Unless defined otherwise, all technical and scientific terms used herein have the same meaning as is commonly understood by one of skill in the art.

As used herein, a malignancy or disease that is associated with FGF receptor expression refers to malignancy, such as melanoma, that in some manner results in a detectable increase in the amount of an FGF receptor in a body fluid, tissue or cell.

As used herein, "high stringency conditions" means conditions of low salt concentration and elevated temperature whereby the oligonucleotide probe for FGFR anneal to the RNA or cDNA encoding FGFR and not non-specifically to other mRNA, i.e., selectively and detectably hybridizes to the RNA or cDNA, whereby stable hybrids form, can be detected, and can be correlated with increased expression of mRNA encoding an FGFR. When performing in situ hybridization reactions, such conditions must not be sufficiently harsh to harm the tissue. High stringency conditions are set forth in Example 2.

As used herein, selectively hybridize means to form hybrids that are sufficiently unique to permit diagnosis of a neoplastic disease. Thus, a probe that selectively hybridizes to DNA encoding a growth factor receptor will form detectable hybrids under the conditions of the assay substantially only with mRNA or cDNA encoding a fibroblast growth factor receptor or with mRNA or cDNA that is substantially homologous to mRNA or cDNA encoding a fibroblast growth factor receptor.

As used herein, a blocker is a composition that prevents non-specific binding of antibodies to the sample tissue. Such blockers include, but are not limited, to such as 3% normal serum from the same species as the second antibody used in the immunoassay; 2% bovine serum albumin or casein; 1.5% normal horse serum or mixtures of any of the preceding compositions. The particular blocker selected and can be empirically determined for each antibody.

### Methods for diagnosing neoplastic diseases

To practice the methods provided herein, a suitable sample is assayed for the presence of a product indicative of elevated levels of an FGF receptor protein and/or expression of a gene encoding an FGF receptor protein. The receptor (hereinafter referred to as an "FGFR") is a cell surface protein receptor with which an FGF specifically interacts. There are numerous subtypes and isoforms of FGFRs, each potentially specific for a particular cell type, but more highly expressed or shed in malignant than in non-malignant tissue. The target product to be assayed can either be an FGFR, a fragment of an FGFR, mRNA or cDNA encoding an FGFR. These products can be detected by any suitable means for detection of specific amino acid or nucleic acid molecules. Preferred for this purpose are immunological techniques for detecting FGFR and nucleic acid hybridization techniques for detecting mRNA encoding FGFRs.

Immunological and hybridization techniques for assaying proteins and mRNA are generally well known in the art and, accordingly, will not be described in detail.

### Immunological methods

Immunological methods are appropriate for detecting FGFRs in body fluids and on cell or tissue surfaces.

### Antigens

An FGF receptor or fragment thereof is an antigen suitable for use in obtaining antibodies useful in the methods. DNA encoding FGF receptors, purified receptors, and methods for obtaining each are known to those of skill in this art. For example, cloning and expression of FGF receptors are described in PCT International Application WO 91/00916 to THE REGENTS OF THE UNIVERSITY OF CALIFORNIA, July 6, 1989, which is based on U.S. Application Serial No. 07/377,033; Isacchi et al. (1990) "Complete Sequence of a Human Receptor for Acidic and Basic Fibroblast Growth Factors", Nucleic Acid Res. 18:1906; Keifer et al., (1991) "Molecular Cloning of A Human Basic FGF Receptor cDNA and Expression of a Biologically Active Extracellular Domain in a Baculovirus System", Growth Factors 05: 115-119; Bergonzoni et al. (1992) "Characterization of a biologically active extracellular domain of fibroblast growth factor receptor 1 expressed in Escherichia coli", Eur. J. Biochem. 210: 823-829; Seno et al. (1991) "Two cDNAs encoding novel human FGF receptor", Biochim. Biophys. Acta 1089:244-246. See, also Johnson et al. (1993) "Structural and Functional Diversity in the FGF Receptor Multigene Family" Advances in Cancer Res. 60:1-41.

Fragments of FGFR may also be used as the antigen for production of either monoclonal or polyclonal antibodies to FGFR(s). One such antigen is the extracellular domain of an FGFR. SEQ ID NO. 1 sets forth DNA and the encoded amino acids of a portion of an extracellular domain of an FGFR 1 . SEQ ID NO. 2 sets forth the amino acid sequence of the entire extracellular domain of an FGFR 1 that has been used as an antigen in the preparation of monoclonal antibodies for use in the immunoassays described herein.

### Antibodies

Antibodies useful in any of these immunological methods can be either polyclonal or monoclonal in nature and the species of origin, e.g., rabbit, goat, rat or murine, is not a critical feature of the methods. The preparation of polyclonal and monoclonal antibodies is well known to those of skill in this art (see, e.g., Goding, J.W. (1986) Monoclonal Antibodies: Principles and Practice, Academic Press, London, NW1 (1986); and Groper, T.G. (1977) The Tools of Biochemistry, John Wiley & Sons, New York). In addition, functional fragments of antibodies such as Fab, (Fab')₂ and peptides having an FGFR binding region can be employed. The means for obtaining such antibodies and fragments are also well known to those skilled in the art and will not be described in detail. Monoclonal antibodies have been prepared using the extracellular domain (SEQ ID NO. 2) of an FGFR 1 as an immunogen. Since portions of the amino acid sequences of extracellular domains are conserved among FGFR1s, monoclonal antibodies raised against this protein should specifically react with the regions of many, if not all, FGFR1 subtypes.

### Immunoassays

The selected antibody or fragment is reacted according to standard immunoassay protocols with serial dilutions of a sample of body fluid, such as urine, blood, a blood fraction or cerebral spinal fluid, or with biopsied tissues. In some instances, mixtures of several antibodies or fragments or antibodies and fragments raised against different FGFR types or subtypes may be used.

### Competitive immunoassays

Immunological techniques that can be used for detecting FGFR in a fluid sample such as blood, serum, cerebral spinal fluid, or urine include, but are not limited to, competitive immunoassays that employ an antibody specific for the receptor. Such assays can also employ a labeled receptor as a second reagent which competes for the captive antibody with receptor present in the sample. The labeled receptor is typically conjugated with a moiety that permits direct or indirect detection. Among the useful labels are radioactive nuclides, such as ³⁵S, ¹²⁵I, ¹³¹I, enzymatic moieties that cause a color change, or luminescence-generating reactions with suitable substrates. Such enzymes include, for example, luciferase, horseradish peroxidase and alkaline phosphatase. Fluorescent labels can also be used. Alternatively, the labeled receptor can be conjugated with a moiety that is recognized by another reagent which permits detection. For example, the receptor can be bound to biotin which will bind to a detectable avidin derivative, e.g., avidin or a derivative or analog therof, such as, streptavidin, conjugated with an enzyme as described above.

### Sandwich immunoassays

Another immunological process useful for detecting FGFR in fluid samples is the two-site or "sandwich" assay. This assay typically uses two antibody preparations, one of which is specific for FGFR and is bound, or capable of being bound, to a solid phase to separate FGFR from other material present in the sample. The second antibody preparation also binds to FGFR at a different site from the first antibody, and is labeled to permit detection of the antibody (solid phase):FGFR:antibody (labeled) complex after the appropriate contacting of the sample with the antibody reagents.

### Direct immunostaining

A third and presently preferred immunological technique for tissue and cell samples, is the direct immunostaining of sections of biopsy material using anti-FGFR antibodies. This is particularly preferred for detection of melanoma. Frozen section or paraffin-fixed sections can be used. The anti-FGFR antibodies can be directly labeled with an enzyme such as peroxidase in order to generate a color reaction when substrate is added to the section. Preferably, however, the section is incubated with the anti-FGFR antibodies, treated with a biotinylated antibody against the species of origin of the primary antibody, and then exposed to a complex of avidin (or streptavidin) biotin and enzyme to increase the amount of enzyme present on the section and thereby increase the sensitivity of the process.

A process for distinguishing melanocytic lesions from non-melanocytic lesions using paraffin-fixed sections of biopsy material and monoclonal or polyclonal antibodies to FGFR has been developed. Using a monoclonal antibody that was specific for the extracellular domain of the FGFR and paraffin-fixed biopsied material, malignant lesions were distinguished from non-malignant lesions, thereby providing a relatively simple staining procedure for diagnosing early stage malignant melanoma.

### Hybridization methods

As pointed out above, mRNA isolated from the cell or tissue that expresses the target receptor gene or cDNA prepared from such mRNA is an alternative to the receptor as a target. Hybridization assays can be performed directly on cells or tissue samples or using mRNA isolated from the cells or tissue or cDNA prepared from the mRNA.

When an assay is performed directly on suspected cancer cells, a nucleic acid probe having a nucleotide sequence complementary to a target mRNA is added to the cells in a manner which permits the probe to selectively hybridize to mRNA in the target tissue or cell that encodes the receptor. Alternatively, mRNA can be isolated from the target cells, tissues or from a body fluid and hybridized with the probe. In addition, cDNA may be prepared from the mRNA and used as a target.

Hybridization reactions are preferably performed under conditions of high stringency, i.e.*,* conditions that select for hybrids that have less than about 10-15% mismatched sequences. Conditions of lower stringency may be used, when the precise identity of the receptor is not known. When hybridizations are performed in situ, however, the conditions must be carefully controlled to avoid harming the tissue or cells. Such conditions, however, can be determined empirically.

### Probes

The probe may be DNA, such as cDNA, or RNA, such as complementary RNA (i.e., antisense RNA) that is prepared from DNA that encodes all or a portion of one or more FGF receptors. The length of the probe can be any length adequate to permit a diagnostic result when the selected stringency hybridization conditions, generally high stringency, are used. Accordingly, the length of the probe is preferably at least about 20 nucleotides, and more preferably, at least about 50 nucleotides, and will not normally exceed about 500 nucleotides. In some instances, it may be advantageous to use mixtures of probes that are complementary to numerous FGFR types and/or subtypes.

Probes or mixtures of probes, based on the DNA sequence of any FGFR or mixtures thereof may be prepared. DNA encoding FGFR subtypes and isoforms of FGFRs is known to those of skill in this art (see, e.g., PCT International Application WO 91/00916 to THE REGENTS OF THE UNIVERSITY OF CALIFORNIA, July 6, 1989, which is based on U.S. Application Serial No. 07/377,033; Isacchi et al., "Complete Sequence of a Human Receptor for Acidic and Basic Fibroblast Growth Factors", Nucleic Acid Res., 18:1906 (1990); Keifer et al. (1991) "Molecular Cloning of A Human Basic FGF Receptor cDNA and Expression of a Biologically Active Extracellular Domain in a Baculovirus System", Growth Factors, 05:115-119; Keegan et al. (1991) "Characterization of the FGFR-3 Gene and its Gene Product", Ann. N.Y. Acad. Sci. 638:400-402); Hou et al. (1991) "Fibroblast Growth Factor Receptors From Liver Vary in Three Structural Domains", Science, 251:665-668 (1991); Partanen et al. (1991) "FGFR-4, A Novel Acidic Fibroblast Growth Factor Receptor With Distinct Expression Pattern", EMBO J., 10:1345-1354; Bergonzoni et al. (1992) "Characterization of a biologically active extracellular domain of fibroblast growth factor receptor 1 expressed in Escherichia coli", Eur. J. Biochem. 210: 823-829; and Seno et al. (1991) "Two cDNAs encoding novel human FGF receptor", Biochim. Biophys. Acta 1089:244-246).

Preferred probes herein include probes that include the sequence of nucleotides or ribonucleotides that have the sequence set forth in SEQ ID NO. 1 or are sufficiently homologous to the DNA of SEQ ID NO. 1 to form stable hybrids under conditions of high stringency.

The probe can be labeled to permit its direct detection. While a variety of labels are useful, since the sample to be assayed will typically be a paraffin-embedded section from a tissue biopsy, it is preferred that the label be one which can generate a color reaction when a suitable substrate is added to the section after any unbound probe has been removed by washing. Among suitable labels, therefore, are enzymes which, in the presence of a suitable substrate, generate a color. These include various peroxidases, such as horseradish peroxidase, alkaline phosphatase and the like.

Although direct labeling can be used, it is preferred to label the probe with biotin and to treat the hybridized probe with an avidin(or streptavidin)/enzyme complex as described above for the immunostaining process. This procedure can conveniently be done by synthesizing the probe using a nucleotide which is biotinylated. Biotinylated uracil triphosphate is preferred for this purpose.

### In situ hybridization assays

A preferred method for determining the presence of elevated levels of FGFR involves in situ hybridization of a probe, for example, a biotinylated cRNA probe, with mRNA in the target tissue in a standard paraffin-fixed section of biopsy material to detect mRNA encoding an FGFR and thereby detect expression of the gene encoding the mRNA. The probe for in situ hybridization is preferably a cRNA copy of DNA that includes a sequence of nucleotides, for example, SEQ ID NO 1, that encodes all or a portion of an FGFR.

The probe is hybridized to a fixed section of a biopsied lesion and the resulting hybrids are detected. In the preferred method in which the hybrids contain biotinylated RNA, the hybrids are treated and stained, as set forth in the Examples. Malignant lesions develop a detectable stain.

### Kits and diagnostic systems

The diagnostic systems may be provided in kit form that is useful for detecting FGFR in body fluid sample. The kit for immunological assays contains antibodies, substantially whole antibodies, or antibody combining sites like Fab and F(ab') sub 2 antibody portions that specifically react with an FGFR in one package. The kit also includes an indicating means for signaling the presence of an immunoreaction between the antibody and FGFR. Typical indicating means include radioisotopes such as ¹²⁵I, ¹³¹I, enzymes, such as alkaline phosphatase, horseradish peroxidase, beta-D-galactosidase and glucose oxidase, and fluorochrome dyes such as fluorescein and rhodamine. The indicating means may be linked directly to the antibody. The indicating means may also be linked to a separate molecule, such as to a second antibody, to an antibody combining site or another entity that binds to an FGF receptor. The indicating means permits the immunoreaction product to be detected, and is packaged separately from the antibody. The kit may also include additional reagents, such as blocking composition to prevent non-specific binding of the labeled antibody used to the sample.

Similarly, kits for performing the hybridization assays herein are provided. Such kits include a probe or mixture of probes that specifically hybridize to one or more FGF receptors. Preferably, such probes will hybridize to the extracellular domain of the receptor. The kit also includes indicating means for detecting the formation of hybrids. As discussed above, the probe is preferably labeled with biotin by synthesizing the probe using a nucleotide, such biotinylated uracil triphosphate. The kit will then also include packaged avidin (or streptavidin)/enzyme complex to treat the resulting hybrids, as described herein, for the immunostaining process.

The antibody molecules, probes and separate indicating means of any diagnostic system described herein, may be provided in solution, as a liquid dispersion or as a substantially dry powder, e.g., in lyophilized form. The indicating means and second antibody or other reagent are packaged separately from the antibody molecules. Where the indicating means is an enzyme, the enzyme's substrate may also be provided in a separate package of the system. A solid support such as the before-described microscope slides and means for paraffin fixing may also be included as separately packaged elements in the diagnostic assay systems. The kits may also include suitable ancillary reagents, such as the appropriate buffers and salts for performing the immunoassays and hybridization reactions.

The packages discussed herein in relation to diagnostic systems are those customarily utilized in diagnostic systems. Such packages include glass and plastic, such as, polyethylene, polypropylene and polycarbonate, bottles, vials, plastic and plastic-foil laminated envelopes and the like.

The following examples are included for illustrative purposes only and are not intended to limit the scope of the invention.

### EXAMPLE 1

### Immunostaining distinguished malignant melanomas from non-malignant lesion

The lesions used in this study were obtained from human biopsy samples. The antibody used in this study was a monoclonal antibody raised against the extracellular domain (SEQ ID NO. 2) of an FGFR 1 (fig 5; see, e.g., Keifer et al. (1991) "Molecular Cloning of A Human Basic FGF Receptor cDNA and Expression of a Biologically Active Extracellular Domain in a Baculovirus System", Growth Factors, 05:115-119). The clinical terminology and staining determinations, set forth below, are known to those of skill in this art (see, e.g., W.F. Lever (1990) Histopathology of the Skin, 7th ed., J.B. Lippincott Company, Philadelphia).

The assays were performed according to the following protocol:
1. Incubate the section in 3% hydrogen peroxide in methanol or phosphate buffered saline (PBS) for three (3) to five (5) minutes (depending on the antibody used) to quench endogenous peroxidase activity.
2. Wash section in phosphate-buffered saline (PBS) containing 0.1-0.5% Triton for 15-30 minutes depending on the antibody used as determined by optimization procedures.
3. Preincubate section for 20 minutes with a 1.5% solution in PBS-0.3% Triton® (sigma, St Louis, MO) of a blocking serum containing non-specific antibody of the same species as the second-labeled antibody (e.g., an antibody of murine origin), to saturate non-specific binding sites reactive with the anti-FGFR antibody.
4. Blot excess serum from the section.
5. Incubate section at 4°C for 16-20 hours with "anti-FGFR antibody solution," prepared as described below.
6. After incubation with anti-FGFR, wash section for fifteen (15) minutes with PBS-0.3% Triton® to remove non-specifically bound antibody.
7. Incubate section with biotinylated anti-mouse antibody (for example, commercially available from Vector Laboratories, Burlingame, CA) diluted in PBS-0.3% Tritoni® containing 1.5% of the blocking serum of step 3 for 45 minutes at room temperature.
8. Wash section with PBS-0.3% Triton® for fifteen (15) minutes.
9. Incubate section for 30 minutes to one (1) hour with avidin conjugated with peroxidase (ABC complex for Vector), diluted in PBS-0.3% Triton ® prepared thirty (30) minutes before use.
10. Wash section with PBS-0.3% Triton® X-100 (the nonionic detergent octoxynol) for fifteen (15) minutes.
11. Incubate section for twenty (20) minutes in peroxidase substrate solution (aminoethylcarbazole from Sigma) in DMSO and 50 mM acetate buffer (pH 5.0), containing 0.01% hydrogen peroxidase.
12. Wash section in deionized water for ten (10) minutes.
13. Counterstain section with hematoxylin (Fisher Chemicals, Tustin, CA) and mount slide with crystal mount.
14. Read section under a microscope comparing color versus lack of color presentation. Red color development indicates a malignant lesion.

As can be seen from Table 1, using the above-described method, malignant melanocytes stained positively. Benign melanocytes, however, stained negatively, thus establishing that expression of an FGF receptor can serve as a diagnostic marker for a neoplasia.

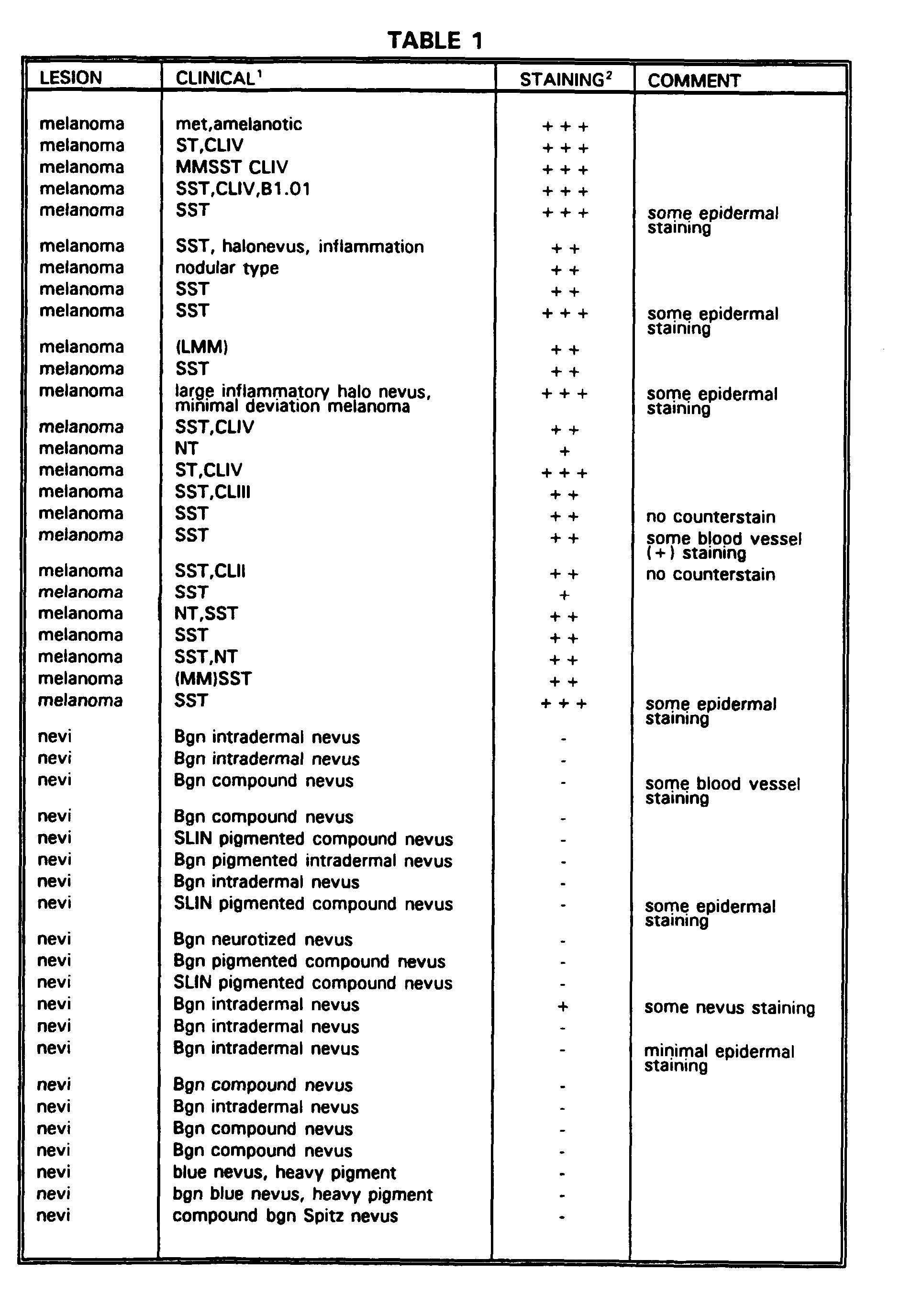

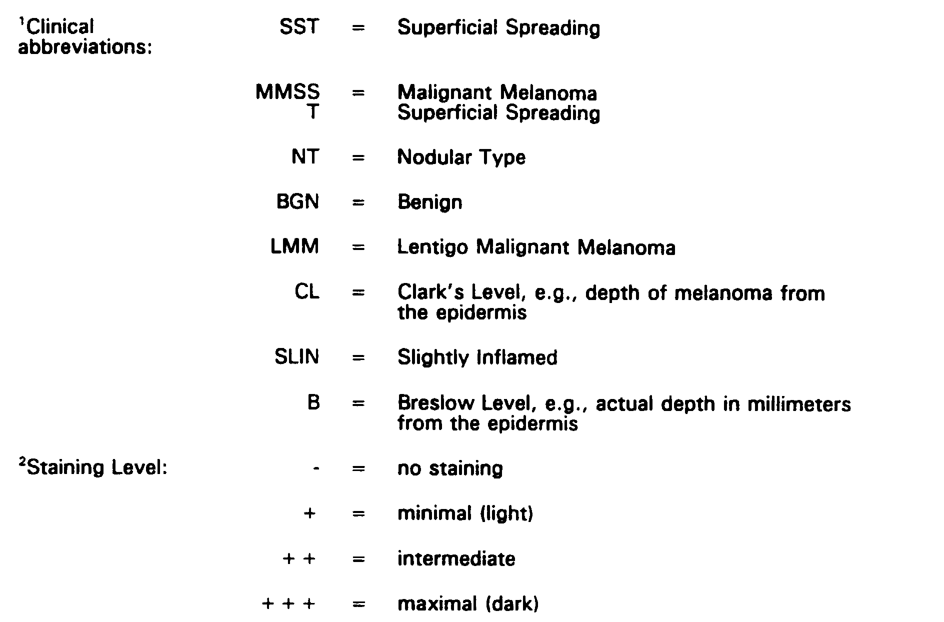

### EXAMPLE 2

### In situ hybridization of FGFR complementary RNA Probe (biotinylated) to mRNA in biopsy samples distinguished malignant melanomas from non-malignant lesions

Lesions were obtained from human biopsy samples and were paraffin fixed on slides. The probe was RNA complementary to the DNA set forth SEQ ID NO. 1. It corresponds to a portion of the extracellular domain of an FGFR that encodes amino acids 158-277 of SEQ ID NO. 1, except that the Gly of the protein of SEQ ID NO. 2 is a Ser (residue 12) in the protein encoded by SEQ ID NO. 1.

The probe was prepared by inserting double-stranded DNA having the sequence set forth in SEQ ID NO. 1 into the plasmid pSP6/T7-19 (LIFE TECHNOLOGIES, INC. Rockville, MD; see, also U.S. Patent Nos. 5,137,829 and 5,026,645). The DNA was inserted into the plasmid such that transcription from the T7 promoter produced cRNA that hybridized to FGFR-encoding mRNA produced in melanocytic tissues. Transcription of opposite strand produced a sense RNA copy which was used as a control.

The assays were performed according to the following protocol:
1. Prior to hybridization, the slide on which the paraffin-fixed section of biopsy material was placed was washed with the following reagents:
   a. xylene (10 min.)
   b. 100% ethanol (10 min.)
   c. air dry (10 min.)
   d. 95% ethanol (2 min.)
   e. 80% ethanol (2 min.)
   f. 70% ethanol (2 min.)
   g. 2 X SSPE buffer 2X (5 min.) (1X SSPE is pH 7.4 sodium phosphate-buffered 0.18 NaCI; see, e.g., Sambrook et al. (1989) Molecular Cloning, A Laboratory Manual, Cold Spring Harbor Laboratory Press (1989), vol. 3, p. B.13,),
   h. Proteinase K (10 µ/ml), 37°C, (30 min.)
   i. 0.1 M TEA (trichloroethylacetic acid), 0.25% Acetic Anhydride; (10 min.)
   j. 2X SSPE buffer, 2X (5 min.)
   k. 0.1 M Tris/glycine pH 7.0 (30 min.0
   l. 2X SSPE buffer, 2X (3 min.)
   m. 70% ethanol (3 min.0
   n. 95% ethanol (3 min.)
   o. air dry (2 hrs.)
2. The cRNA probe was diluted to 800 ng/ml with buffer and was applied to slide. The coverslips were placed on the slide; sealed and the slide was incubated overnight at 55°C on a warm plate. The buffer contained the following ingredients: 5 ml deionized formamide; 2 ml dextran sulfate; 2 ml SSPE (20X); 0.2 ml of 50X Denhardt's reagent (50X Denhardt's reagent contains 5 g Ficoll (Type 400, PHARMACIA FINE CHEMICALS, Uppsala, Sweden), 5g polyvinylpyrrolidone, 5 g bovine serum albumin (Fraction V; SIGMA, Madison, WI), and H₂O to 500 ml); 0.5 ml total RNA (salmon sperm (10 µg/ml)); and 0.25 ml yeast tRNA (10 µg/ml).
3. The slide was rinsed with 4X SSPE for 20 minutes to remove the coverslip followed by 3X 10-minute rinses and the section was then rinsed as follows:.
   a. 50% formamide/2X SSPE, 2X for 15 minutes at 48°-50°C.
   b. 2X SSPE buffer, 2X (10 min.)
4. The section was incubated with RNaseA (10 µg/ml), 37°C (30 min.) and rinsed as follows:
   a. 2X SSPE buffer, 2X (10 min).
   b. 50% formamide/2X SSPE at 48°-50°C (15 min.)
   c. 2X SSPE buffer (5 min.)
   d. 0.1 M Tris Saline (5 min.)
5. The section was incubated with avidin complex with alkaline phosphatase (Vector) for 1 hour at room temperature and then rinsed with 0.1 M Tris saline for 5 minutes (3X).
6. The section was incubated with Fast Red solution for 8 hours (optionally with Levamisole, an inhibitor of endogenous alkaline phosphatase) and then rinsed with deionized water 3X.
7. The section was then counterstained with hematoxylin (optional) and rinsed with deionized water.
8. The section was mounted with crystal mount and read under a microscope. Color development indicates that the sample was a malignant lesion.

As shown in Table 2, malignant melanocytes stain positively using the techniques described above. Benign melanocytes stain negatively using the same techniques, thus establishing that there is increased expression of the mRNA in neoplastic cells that hybridizes to DNA encoding a portion of an FGF receptor.

**TABLE 2**

| LESION | CLINICAL¹ | STAINING² | COMMENT |
|---|---|---|---|
| melanoma | SST | ++ | |
| melanoma | SST | ++ | grainy |
| melanoma | SST | ++ | |
| nevi (intradermal) | | - | |
| nevi (compound) | | - | |
| nevi (intradermal) | | - | some epidermal staining |

| | | | |
|---|---|---|---|
| ¹Clinical Abbreviations SST = Superficial Spreading - = no staining + = minimal (light) + + = intermediate + + + = maximal (dark) | | | |
| ²Staining Level | | | |

## Claims

1. A process for detecting a neoplastic disease in a subject, comprising detecting, in a tissue or body fluid sample obtained from the subject, elevated expression of a fibroblast growth factor receptor (FGFR) gene or elevated amounts of (fibroblast growth factor receptor) FGFR protein, wherein:
the amounts or expression are elevated compared to the amounts or expression in a sample from a subject in whom the neoplastic disease is not present; and
detection of the elevated amounts or expression indicates the presence of a neoplastic disease in the subject.

2. The process of claim 1, wherein detection is accomplished by:
(a) contacting a sample obtained from said subject with an agent that specifically binds to either FGFR or to nucleic acid encoding FGFR; and
(b) detecting any binding of said agent to the FGFR or nucleic acid, wherein elevated amount binding indicates the presence of a neoplastic disease.

3. The process of claim 2, wherein said agent is an antibody or a functional fragment thereof.

4. The process of claim 2, wherein said agent is a nucleic acid probe.

5. The process of claim 2, wherein said agent is labelled with a detectable marker.

6. The process of any of claims 1-5, wherein the FGFR protein is detected.

7. The process of any of claims 1, 2, 5 and 6, wherein the process is an immunoassay.

8. The process of claim 7, comprising: treating a paraffin-fixed section of biopsy material with an antibody having specific reactivity with the receptor protein, removing unbound antibody from the section and detecting the antibody bound to receptor protein present in the section, wherein the detection of antibody bound to said receptor protein indicates the presence of neoplastic disease in said subject.

9. The process of claim 8, wherein the bound antibody is detected by:
(a) incubating the section with a second antibody that binds to the first antibody, wherein said second antibody is detectably labelled;
(b) removing unbound second antibody from the section; and
(c) detecting complexes containing the second antibody.

10. The process of any of claims 1, 2 and 4-6, wherein mRNA or cDNA that encodes the FGFR protein is detected.

11. A process according to claim 10, wherein the mRNA or cDNA is jetected by hybridization with a detectable nucleic acid probe having a nucleotide sequence sufficiently complementary to at least a portion of the mRNA or cDNA to hybridize thereto.

12. The process of claim 4 or claim 11, wherein the probe is bio inylated.

13. The process of claim 4 or claim 11, wherein the probe is labeled with digoxin.

14. The process of any of claims 11-13, wherein hybridization of the probe to mRNA in the sample is detected by:
(a) removal of unbound probe from the section;
(b) incubation of the section with a complex of avidin or streptavidin and alkaline phosphatase;
(c) removal of unbound avidin or streptavidin/alkaline phosphatase complex from the section;
(d) treatment of the section with a color forming substrate for alkaline phosphatase;
(e) removal of unbound substrate; and
(f) reading the section to determine the presence of neoplastic disease.

15. The method of claim 9, wherein the second antibody is biotinylated; and following removal of unbound second antibody the section is incubated with a complex of avidin or streptavidin and peroxidase; the unbound avidin or streptavidin/peroxidase complex is removed; the section is treated with a color forming substrate for peroxidase; the unbound substrate is removed; and complexes are detected by reading the section to detect any color change to determine the presence of neoplastic disease.

16. The process of any of claims 1-15, wherein the neoplastic disease is malignant melanoma.

17. The process of any of claims 4 or 10-16, wherein the probe includes nucleotides or ribonucleotides that have the sequence set forth in SEQ ID NO. 1 or are sufficiently homologous to the DNA of SEQ ID NO. 1 to form stable hybrids under conditions of high stringency.

18. The process of any of claims 1-7, 10-14, 16 and 17, wherein the sample is blood, urine, a blood fraction or cerebral spinal fluid.

19. The process of any of claims 1-17, wherein the sample is biopsied tissue.

20. A kit for diagnosing neoplastic disease, comprising:
(a) a first antibody that specifically reacts with one or more fibroblast growth factor receptors (FGFRs);
(b) indicating means for detecting immunoreaction between the antibody and an FGFR;
(c) reagents for performing direct immunostaining of biopsied tissue samples.

21. A kit for diagnosing neoplastic disease, comprising:
(a) one or more nucleic acid probes that selectively hybridize to one or more mRNA or cDNA encoding human fibroblast growth factor receptors (FGFRs);
(b) indicating means for detecting hybrids between the probes and mRNA or cDNA encoding all or a detectable portion of an FGFR; and
(b) reagents for in situ hybridization with cell or tissue samples.

22. The kit of claim 20 or 21, wherein the neoplastic disease is malignant melanoma.

23. The kit of claim 20 or 22, wherein the antibody specifically binds to the extracellular domain of a fibroblast growth factor receptor.

24. The kit of claim 20, 22 or 23, 20 or 21, further comprising a second antibody that specifically binds to the first antibody.

25. The kit of claim 21 or 22, wherein the probe specifically hybridizes to DNA encoding the extracellular domain of a fibroblast growth factor receptor.

26. The kit of any of claims 20, 23 and 24, wherein the probe includes nucleotides or ribonucleotides that have the sequence set forth in SEQ ID NO. 1 or are sufficiently homologous to the DNA of SEQ ID NO. 1 to form stable hybrids under conditions of high stringency.

27. The kit of any of claims 20, 22, 23 or 24 wherein the reagents include a blocker that prevents non-specific binding of the antibody to the tissue sample.

28. The kit of any of claims 20, 22-24 and 27, wherein the antibody specifically reacts with the protein having SEQ ID NO. 2 or the protein encoded by SEQ ID NO. 1.

## Patentansprüche

1. Verfahren zum Nachweis einer neoplastischen Erkrankung in einem Individuum, das den Nachweis einer gesteigerten Expression eines Fibroblasten-Wachstumsfaktor-Rezeptor (FGFR)-Gens oder erhöhter Mengen eines (Fibroblasten-Wachstumsfaktor-Rezeptor-) FGFR-Proteins in einer Gewebe- oder Körperflüssigkeits-Probe, die von dem Individuum erhalten wurde, umfaßt, worin:
die Mengen oder die Expression erhöht sind im Vergleich mit den Mengen oder der Expression in einer Probe von einem Individuum, bei dem keine neoplastische Erkrankung vorliegt; und
der Nachweis der erhöhten Mengen oder Expression die Gegenwart einer neoplastischen Erkrankung in dem Individuum anzeigt.

2. Verfahren nach Anspruch 1, bei dem der Nachweis dadurch bewirkt wird, daß man:
(a) eine Probe, die von dem genannten Individuum erhalten wurde, mit einem Mittel umsetzt, das spezifisch entweder an FGFR oder die Nukleinsäure bindet, die für FGFR codiert; und
(b) jegliche Bindung des genannten Mittels an FGFR oder die Nukleinsäure nachweist, wobei eine erhöhte mengenmäßige Bindung das Vorliegen einer neoplastischen Erkrankung anzeigt.

3. Verfahren nach Anspruch 2, bei dem das genannte Mittel ein Antikörper oder ein funktionelles Fragment davon ist.

4. Verfahren nach Anspruch 2, bei dem das genannte Mittel eine Nukleinsäure-Sonde ist.

5. Verfahren nach Anspruch 2, bei dem das genannte Mittel mit einer nachweisbaren Markierung markiert ist.

6. Verfahren nach irgendeinem der Ansprüche 1 bis 5, bei dem das FGFR-Protein nachgewiesen wird.

7. Verfahren nach irgendeinem der Ansprüche 1, 2, 5 und 6, wobei das Verfahren ein Immunoassay ist.

8. Verfahren nach Anspruch 7, das umfaßt: Behandeln eines paraffinfixierten Schnitts eines Biopsie-Materials mit einem Antikörper, der eine spezifische Reaktivität gegenüber dem Rezeptorprotein aufweist, Entfernen von ungebundenem Antikörper von dem Schnitt und Nachweisen des Antikörpers, der an das Rezeptorprotein gebunden ist, das in dem Schnitt vorhanden ist, wobei der Nachweis von Antikörper, der an das genannte Rezeptorprotein gebunden ist, das Vorliegen einer neoplastischen Erkrankung in dem genannten Individuum anzeigt.

9. Verfahren nach Anspruch 8, bei dem der gebundene Antikörper nachgewiesen wird durch:
(a) Inkubieren des Schnitts mit einem zweiten Antikörper, der an den ersten Antikörper bindet, wobei der zweite Antikörper nachweisbar markiert ist;
(b) Entfernen des ungebundenen zweiten Antikörpers von dem Schnitt; und
(c) Nachweisen von Komplexen, die den zweiten Antikörper enthalten.

10. Verfahren nach irgendeinem der Ansprüche 1, 2 und 4 bis 6, bei dem mRNA oder cDNA, die für das FGFR-Protein codiert, nachgewiesen wird.

11. Verfahren nach Anspruch 10, bei dem die mRNA oder cDNA durch Hybridisierung mit einer nachweisbaren Nukleinsäure-Sonde nachgewiesen wird, die eine Nukleotidsequenz aufweist, die ausreichend komplementär zu wenigstens einem Teil der mRNA oder cDNA ist, so daß sie damit hybridisiert.

12. Verfahren nach Anspruch 4 oder Anspruch 11, bei dem die Sonde biotinyliert ist.

13. Verfahren nach Anspruch 4 oder Anspruch 11, bei dem die Sonde mit Digoxin markiert ist.

14. Verfahren nach irgendeinem der Ansprüche 11 bis 13, bei dem die Hybridisierung der Sonde mit mRNA in der Probe nachgewiesen wird durch:
(a) Entfernung ungebundener Sonde von dem Schnitt;
(b) Inkubation des Schnitts mit einem Komplex aus Avidin oder Streptavidin und alkalischer Phosphatase;
(c) Entfernung von ungebundenem Komplex aus Avidin oder Streptavidin und alkalischer Phosphatase von dem Schnitt;
(d) Behandlung des Schnitts mit einem farbbildenden Substrat für alkalische Phosphatase;
(e) Entfernung von ungebundenem Substrat; und
(f) Auswertung des Schnitts, um das Vorliegen einer neoplastischen Erkrankung zu bestimmen.

15. Verfahren nach Anspruch 9, bei dem der zweite Antikörper biotinyliert ist und bei dem im Anschluß an die Entfernung von ungebundenem zweitem Antikörper der Schnitt mit einem Komplex aus Avidin oder Streptavidin und Peroxidase inkubiert wird; der ungebundene Avidin- oder Streptavidin-Peroxidase-Komplex entfernt wird; der Schnitt mit einem farbbildenden Substrat für Peroxidase behandelt wird; das ungebundene Substrat entfernt wird; und Komplexe dadurch nachgewiesen werden, daß man den Schnitt auswertet, um jegliche Farbveränderung nachzuweisen, um das Vorliegen einer neoplastischen Erkrankung zu bestimmen.

16. Verfahren nach irgendeinem der Ansprüche 1 bis 15, bei dem die neoplastische Erkrankung ein malignes Melanom ist.

17. Verfahren nach irgendeinem der Ansprüche 4 oder 10 bis 16, bei dem die Sonde Nukleotide oder Ribonukleotide einschließt, die die Sequenz aufweisen, die in SEQ ID Nr. 1 gezeigt ist oder die zu der DNA von SEQ ID Nr. 1 ausreichend homolog ist, so daß unter Bedingungen einer hohen Stringenz stabile Hybride gebildet werden.

18. Verfahren nach irgendeinem der Ansprüche 1 bis 7, 10 bis 14, 16 und 17, bei dem die Probe Blut, Urin, eine Blutfraktion oder cerebrales spinales Fluid ist.

19. Verfahren nach irgendeinem der Ansprüche 1 bis 17, bei dem die Probe ein Biopsie-Gewebe ist.

20. Reagenziensatz für die Diagnose einer neoplastischen Erkrankung, der aufweist:
(a) einen ersten Antikörper, der spezifisch mit einem oder mehreren Fibroblasten-Wachstumsfaktor-Rezeptoren (FGFRs) reagiert;
(b) Anzeigemittel zum Nachweis einer Immunreaktion zwischen dem Antikörper und einem FGFR;
(c) Reagenzien zur Durchführung einer direkten Immunanfärbung von Biopsie-Gewebeproben.

21. Reagenziensatz für die Diagnose einer neoplastischen Erkrankung, der aufweist:
(a) eine oder mehrere Nukleinsäure-Sonde(n), die selektiv mit einer oder mehreren mRNA oder cDNA hybridisieren, die für humane Fibroblasten-Wachstumsfaktor-Rezeptoren (FGFRs) codieren;
(b) Anzeigemittel für den Nachweis von Hybriden zwischen den Sonden und mRNA oder cDNA, die für einen vollständigen oder einen nachweisbaren Teil eines FGFR codieren; und
(c) Reagenzien für die in-Situ-Hybridisierung mit Zell- oder Gewebeproben.

22. Reagenziensatz nach Anspruch 20 oder 21, bei dem die neoplastische Erkrankung ein malignes Melanom ist.

23. Reagenziensatz nach Anspruch 20 oder 22, bei dem der Antikörper spezifisch an die extrazelluläre Domäne eines Fibroblasten-Wachstumsfaktor-Rezeptors bindet.

24. Reagenziensatz nach Anspruch 20, 22 oder 23, der außerdem einen zweiten Antikörper umfaßt, der spezifisch an den ersten Antikörper bindet.

25. Reagenziensatz nach Anspruch 21 oder 22, bei dem die Sonde spezifisch mit DNA hybridisiert, die für die extrazelluläre Domäne eines Fibroblasten-Wachstumsfaktor-Rezeptors codiert.

26. Reagenziensatz nach irgendeinem der Ansprüche 20, 23 und 24, bei dem die Sonde Nukleotide oder Ribonukleotide aufweist, die die Sequenz aufweisen, die in SEQ ID Nr. 1 gezeigt ist, oder die zu der DNA von SEQ ID Nr. 1 ausreichend homolog sind, um unter Bedingungen einer hohen Stringenz stabile Hybride zu bilden.

27. Reagenziensatz nach irgendeinem der Ansprüche 20, 22, 23 oder 24, worin die Reagenzien einen Blocker einschließen, der ein nicht-spezifisches Binden des Antikörpers an die Gewebeprobe verhindert.

28. Reagenziensatz nach irgendeinem der Ansprüche 20, 22 bis 24 und 27, worin der Antikörper spezifisch mit dem Protein reagiert, das die Sequenz SEQ ID Nr. 2 aufweist, oder mit dem Protein, für das SEQ ID Nr. 1 codiert.

## Revendications

1. Procédé de détection d'une maladie néoplasique chez un sujet, comprenant la détection dans un échantillon tissulaire ou de fluide corporel prélevé sur le sujet, d'une élévation du taux d'expression d'un gène codant pour le récepteur au facteur de croissance des fibroblastes (FGFR) ou de quantités élevées de protéine FGFR (récepteur au facteur de croissance des fibroblastes), dans lequel :
le taux d'expression ou les quantités sont élevées par rapport aux quantités et au taux d'expression trouvé pour un échantillon prélevé sur un sujet non atteint de maladie néoplasique; et
la détection de taux d'expression ou de quantités plus élevées indique l'atteinte du sujet par une maladie néoplasique.

2. Procédé selon la revendication 1, dans lequel la détection s'effectue par
(a) la mise en contact d'un échantillon prélevé sur ledit sujet avec un agent qui se lie de manière spécifique soit au FGFR soit à l'acide nucléique codant pour le FGFR; et
(b) la détection de toute liaison dudit agent au FGFR ou à l'acide nucléique, procédé dans lequel un degré de fixation plus élevé indique la présence d'une maladie néoplasique.

3. Procédé selon la revendication 2, dans lequel ledit agent est un anticorps ou un fragment fonctionnel de celui-ci.

4. Procédé selon la revendication 2, dans lequel ledit agent est une sonde à base d'acide nucléique.

5. Procédé selon la revendication 2, dans lequel ledit agent est marqué avec un marqueur détectable.

6. Procédé selon l'une quelconque des revendications 1 à 5, dans lequel la protéine FGFR est détectée.

7. Procédé selon l'une quelconque des revendications 1, 2, 5 et 6, dans lequel le procédé consiste en un dosage immunologique.

8. Procédé selon la revendication 7, comprenant le traitement d'une coupe d'un échantillon de biopsie fixée et imprégnée à la paraffine avec un anticorps donnant une réaction spécifique avec la protéine récepteur, l'élimination de l'anticorps non lié de la coupe et la détection de l'anticorps lié à la protéine récepteur présente dans la coupe, procédé dans lequel la détection d'anticorps lié à ladite protéine récepteur indique la présence de maladies néoplasiques chez ledit sujet.

9. Procédé selon la revendication 8, dans lequel l'anticorps lié est détecté par:
(a) la mise en incubation de la coupe avec un deuxième anticorps qui se lie au premier anticorps, dans lequel le deuxième anticorps porte un marqueur détectable;
(b) l'élimination du deuxième anticorps non lié de la coupe; et
(c) la détection de complexes contenant le deuxième anticorps.

10. Procédé selon l'une quelconque des revendications 1, 2 et 4 à 6, dans lequel il y a détection de l'ARNm ou l'ADNc qui code pour la protéine FGFR.

11. Procédé selon la revendication 10, dans lequel l'ARNm ou l'ADNc est détecté par hybridation avec une sonde d'acide nucléique détectable ayant une séquence nucléotidique suffisamment complémentaire à au moins une partie de l'ARNm ou de l'ADNc pour qu'elle s'y hybride.

12. Procédé selon la revendication 4 ou la revendication il, dans lequel la sonde est biotinylée.

13. Procédé selon la revendication 4 ou la revendication 11, dans lequel la sonde est marquée par la digoxine.

14. Procédé selon l'une quelconque des revendications Il à 13, dans lequel l'hybridation de la sonde à l'ARNm dans l'échantillon est détectée par:
(a) l'élimination de la sonde non liée de la coupe;
(b) la mise en incubation de la coupe avec un complexe associant l'avidine ou la streptavidine à la phosphatase alcaline.
(c) l'élimination du complexe avidine ou streptavidine/phosphatase alcaline non lié de la coupe;
(d) le traitement de la coupe avec un substrat chromogène de la phosphatase alcaline;
(e) l'élimination du substrat non lié; et
(f) l'examen de la coupe pour déterminer la présence de maladie néoplasique.

15. Procédé selon la revendication 9, dans lequel le second anticorps est biotinylé; et suite à l'élimination du deuxième anticorps non lié, la coupe est mise à incubée avec un complexe associant l'avidine ou la streptavidine à la peroxydase; le complexe avidine ou streptavidine/peroxydase non lié est éliminé; la coupe est traitée avec un substrat chromogène de la peroxydase; le substrat non lié est éliminé; et la formation de complexes est détectée en examinant la coupe en vue de déceler tout virage de couleur et déterminer ainsi la présence de maladies néoplasiques.

16. Procédé selon l'une quelconque des revendications 1 à 15, dans lequel la maladie néoplasique est le mélanome malin.

17. Procédé selon l'une quelconque des revendications 4 ou 10 à 16, dans lequel la sonde comprend des nucléotides ou des ribonucléotides répondant à la séquence portant le N° d'identification 1 ou sont suffisamment homologues à l'ADN de la séquence portant le N° d'identification 1 pour former des hybrides stables en milieu de forte stringence.

18. Procédé selon l'une quelconque des revendications 1 à 7, 10 à 14, 16 et 17, dans lequel l'échantillon est du sang, de l'urine, un dérivé du sang ou du liquide céphalo-rachidien.

19. Procédé selon l'une quelconque des revendications 1 à 17, dans lequel l'échantillon est un tissu prélevé par biopsie.

20. Trousse pour diagnostiquer une maladie néoplasique, comprenant :
(a) un premier anticorps qui réagit de manière spécifique avec un ou plusieurs récepteurs au facteur de croissance des fibroblastes (FGFR):
(b) des moyens indicateurs pour détecter la réaction immunologique entre l'anticorps et un FGFR;
(c) des réactifs pour réaliser une coloration immunohistologique directe des échantillon tissulaires prélevés par biopsie.

21. Trousse destinée au diagnostic de maladies néoplasiques, comprenant :
(a) une ou plusieurs sondes d'acide nucléique qui s'hybrident de manière sélective à un ou plusieurs ARNm ou ADNc codant pour les récepteurs du facteur de croissance des fibroblastes humain (FGFR);
(b) des moyens indicateurs pour détecter la formation d'hybrides entre les sondes et l'ARNm ou l'ADNc codant pour l'intégralité ou une partie détectable d'un FGFR; et
(c) des réactifs pour réaliser une hybridation *in situ* sur des échantillons cellulaires ou tissulaires.

22. Trousse selon la revendication 20 ou 21, dans laquelle la maladie néoplasique est le mélanome malin.

23. Trousse selon la revendication 20 ou 22, dans laquelle l'anticorps se lie de manière spécifique au domaine extracellulaire d'un récepteur au facteur de croissance des fibroblastes.

24. Trousse selon la revendication 20, 22, ou 23, comprenant en outre un deuxième anticorps qui se lie de manière spécifique au premier anticorps.

25. Trousse selon la revendication 21 ou 22, dans laquelle la sonde s'hybride de manière spécifique à l'ADN codant pour le domaine extracellulaire d'un récepteur au facteur de croissance des fibroblastes.

26. Trousse selon l'une quelconque des revendications 20, 23 et 24, dans laquelle la sonde comprend des nucléotides ou des ribonucléotides qui répondent à la Séquence portant le N° d'identification 1 ou sont suffisamment homologues à l'ADN correspondant à la Séquence portant le N° d'identification 1 pour former des hybrides stables en milieu de forte stringence.

27. Trousse selon l'une quelconque des revendications 20, 22, 23 ou 24, dans laquelle les réactifs comprennent un agent de passivation qui prévient la fixation non spécifique de l'anticorps à l'échantillon tissulaire.

28. Trousse selon l'une quelconque des revendications 20, 22, 24 et 27, dans laquelle l'anticorps réagit de manière spécifique avec la protéine répondant à la séquence portant le N° d'identification 2 ou la protéine encodée par la séquence portant le N° d'identification 1.


### SEQUENCE LISTING

(1) GENERAL INFORMATION:
   (i) APPLICANT: Nova, Michael P. Gonzalez, Ana-Maria Baird, Andrew
   (ii) TITLE OF INVENTION: Process for Detection of Neoplastic Disease
   (iii) NUMBER OF SEQUENCES: 2
   (iv) CORRESPONDENCE ADDRESS:
      (A) ADDRESSEE: Fitch, Even, Tabin & Flannery
      (B) STREET: 135 South LaSalle Street, Suite 900
      (C) CITY: Chicago
      (D) STATE: IL
      (E) COUNTRY: USA
      (F) ZIP: 60603
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 07/900,646
      (B) FILING DATE: 18-JUN-1992
   (viii) ATTORNEY/AGENT INFORMATION:
      (A) NAME: Seidman, Stephanie
      (B) REGISTRATION NUMBER: 33,779
      (C) REFERENCE/DOCKET NUMBER: 54625PCT
   (ix) TELECOMMUNICATION INFORMATION:
      (A) TELEPHONE: 619-552-1311
      (B) TELEFAX: 619-552-0095
(2) INFORMATION FOR SEQ ID NO:1:
   (i) SEQUENCE CHARACTERISTICS:
      (A) LENGTH: 360 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION: 1..360
      (D) OTHER INFORMATION: /standard underscores names= ""flg probe""
      (xi) SEQUENCE DESCRIPTION: SEQ ID NO:1:
   ACC CCA AAC CCC ACA CTG CGC TGG TTG AAA AAT AGC AAA GAA TTC AAA      48
   Thr Pro Asn Pro Thr Leu Arg Trp Leu Lys Asn Ser Lys Glu Phe Lys
    1               5                  10                  15
   CCT GAC CAC AGA ATT GGA GGC TAC AAG GTC CGT TAT GCC ACC TGG AGC      96
   Pro Asp His Arg Ile Gly Gly Tyr Lys Val Arg Tyr Ala Thr Trp Ser
               20                  25                  30
   ATC ATA ATG GAC TCT GTG GTG CCC TCT GAC AAG GGC AAC TAC ACC TGC     144
   Ile Ile Met Asp Ser Val Val Pro Ser Asp Lys Gly Asn Tyr Thr Cys
           35                  40                  45
   ATT GTG GAG AAT GAG TAC GGC AGC ATC AAC CAC ACA TAC CAG CTG GAT     192
   Ile Val Glu Asn Glu Tyr Gly Ser Ile Asn His Thr Tyr Gln Leu Asp
       50                  55                  60
   GTC GTG GAG CGG TCC CCT CAC CGG CCC ATC CTG CAA GCA GGG TTG CCC     240
   Val Val Glu Arg Ser Pro His Arg Pro Ile Leu Gln Ala Gly Leu Pro
   65                  70                  75                  80
   GCC AAC AAA ACA GTG GCC CTG GGT AGC AAC GTG GAG TTC ATG TGT AAG     288
   Ala Asn Lys Thr Val Ala Leu Gly Ser Asn Val Glu Phe Met Cys Lys
                   85                  90                  95
   GTG TAC AGT GAC CCG CAG CCG CAC ATC CAG TGG CTA AAG CAC ATC GAG     336
   Val Tyr Ser Asp Pro Gln Pro His Ile Gln Trp Leu Lys His Ile Glu
               100                 105                 110
   GTG AAT GGG AGC AAG ATT GGC CCA                                     360
   Val Asn Gly Ser Lys Ile Gly Pro
           115                 120
(2) INFORMATION FOR SEQ ID NO:2:
   (i) SEQUENCE CHARACTERISTICS:
      (A) LENGTH: 351 amino acids
      (B) TYPE: amino acid
      (C) STRANDEDNESS: single
      (D) TOPOLOGY: unknown
   (ii) MOLECULE TYPE: protein
   (ix) FEATURE:
      (A) NAME/KEY: Protein
      (B) LOCATION: 1..351
      (D) OTHER INFORMATION: /note= "Sequence of extracellular domain of an FGFR1"
   (xi) SEQUENCE DESCRIPTION: SEQ ID NO:2:
   Ser Pro Thr Leu Pro Glu Gln Ala Gln Pro Trp Gly Ala Pro Val Glu
   1               5                   10                  15
   Val Glu Ser Phe Leu Val His Pro Gly Asp Leu Leu Gln Leu Arg Cys
               20                  25                  30
   Arg Leu Arg Asp Asp Val Gln Ser Ile Asn Trp Leu Arg Asp Gly Val
           35                  40                  45
   Gln Leu Ala Glu Ser Asn Arg Thr Arg Ile Thr Gly Glu Glu Val Glu
       50                  55                  60
   Val Gln Asp Ser Val Pro Ala Asp Ser Gly Leu Tyr Ala Cys Val Thr
   65 70 75 80
   Ser Ser Pro Ser Gly Ser Asp Thr Thr Tyr Phe Ser Val Asn Val Ser
   85 90 95
   Asp Ala Leu Pro Ser Ser Glu Asp Asp Asp Asp Asp Asp Asp Ser Ser
   100 105 110
   Ser Glu Glu Lys Glu Thr Asp Asn Thr Lys Pro Asn Pro Val Ala Pro
   115 120 125
   Tyr Trp Thr Ser Pro Glu Lys Met Glu Lys Lys Leu His Ala Val Pro
   130 135 140
   Ala Ala Lys Thr Val Lys Phe Lys Cys Pro Ser Ser Gly Thr Pro Asn
   145 150 155 160
   Pro Thr Leu Arg Trp Leu Lys Asn Gly Lys Glu Phe Lys Pro Asp His
   165 170 175
   Arg Ile Gly Gly Tyr Lys Val Arg Tyr Ala Thr Trp Ser Ile Ile Met
   180 185 190
   Asp Ser Val Val Pro Ser Asp Lys Gly Asn Tyr Thr Cys Ile Val Glu
   195 200 205
   Asn Glu Tyr Gly Ser Ile Asn His Thr Tyr Gln Leu Asp Val Val Glu
   210 215 220
   Arg Ser Pro His Arg Pro Ile Leu Gln Ala Gly Leu Pro Ala Asn Lys
   225 230 235 240
   Thr Val Ala Leu Gly Ser Asn Val Glu Phe Met Cys Lys Val Tyr Ser
   245 250 255
   Asp Pro Gln Pro His Ile Gln Trp Leu Lys His Ile Glu Val Asn Gly
   260 265 270
   Ser Lys Ile Gly Pro Asp Asn Leu Pro Tyr Val Gln Ile Leu Lys Thr
   275 280 285
   Ala Gly Val Asn Thr Thr Asp Lys Glu Met Glu Val Leu His Leu Arg
   290 295 300
   Asn Val Ser Phe Glu Asp Ala Gly Glu Tyr Thr Cys Leu Ala Gly Asn
   305 310 315 320
   Ser Ile Gly Leu Ser His His Ser Ala Trp Leu Thr Val Leu Glu Ala
   325 330 335
   Leu Glu Glu Arg Pro Ala Val Met Thr Ser Pro Leu Tyr Leu Glu
   340 345 350